# EUROPEAN PATENT SPECIFICATION

(11) **EP 4 119 038 B1**
(45) Date of publication and mention of the grant of the patent: **09.12.2026**
(21) Application number: 21185093.8
(22) Date of filing: 12.07.2021
(51) Int. Cl.: A61B 5/00, A61B 5/08

(54) **METHOD FOR QUANTIFICATION OF A LEVEL OF RESPONSE TO HYPOGLOSSAL NERVE STIMULATION**
VERFAHREN ZUR QUANTIFIZIERUNG DES REAKTIONSGRADES AUF DIE STIMULATION DES HYPOGLOSSALEN NERVS
PROCÉDÉ DE QUANTIFICATION D'UN NIVEAU DE LA RÉPONSE À UNE STIMULATION DU NERF GRAND HYPOGLOSSE

(43) Date of publication of application: 18.01.2023
(73) Proprietor: Nyxoah SA, 1435 Mont-St-Guibert (BE)
(72) Inventor: KOHN, Sarah, Modiin 7174661 (IL); KATZIR, Doron, Ramat-Gan 5253212 (IL); TSUKRAN, Roi, Rishon Le-Zion 7575721 (IL); TOBERMAN, Daniel, Jerusalem 9371211 (IL); LICHTENSTADT, Assaf, Petah Tikva 4974355 (IL); NELSON, Dwight, Shoreview, MN 55126 (US); RAUX, Guillaume, 1310 La Hulpe (BE)
(74) Representative: Brantsandpatents bv

(56) References cited:
- US-A1- 2014 228 905

## Description

### Technical Field

The disclosed subject-matter described hereafter refers to a method for quantification of response level to nerve stimulation. In particular, the subject-matter refers to method for quantification of response level to nerve stimulation in the treatment of obstructive sleep apnea (OSA).

### Background

Neural modulation, e.g. electrical stimulation of nerves, is -known in the prior art as a reliable and effective type of medical treatment. It presents the opportunity to tackle many physiological conditions and disorders by interacting with the body's own natural neural processes. Neural modulation includes inhibition (e.g. blockage), stimulation, modification, regulation, or therapeutic alteration of activity, electrical or chemical, in the central, peripheral, or autonomic nervous system. By modulating the activity of the nervous system, several different goals may be achieved. For instance, motor neurons may be stimulated at appropriate times to cause muscle contractions. Further, sensory neurons can be blocked to relieve pain or stimulated to provide a signal to a subject. In yet other examples, modulation of the autonomy nervous system may be used to adjust various involuntary physiological parameters, such as heart rate and blood pressure. Neural modulation may provide the opportunity to treat several diseases or physiological conditions. Various devices and techniques have been used in attempts to provide optimum stimulation of a tissue of interest.

Within the meaning of this disclosure, the terms "stimulation", "modulation", "neurostimulation" and "neuromodulation" are used synonymously, unless something else is apparent from any given context.

Typically, neural stimulators deliver therapy in the form of electrical pulses and include one or more electrode in a proximity of the target location, such as a specific nerve or section thereof. Electrical stimulation is programmable and adjustable through various parameters, such as the polarity of electrode(s), voltage, current amplitudes, pulse frequency, pulse width, and others as these define the electrical stimulation therapy to be delivered to the user in need of therapy. Such parameters may be preprogrammed or programmable to deliver the desired stimulation and end result desired from the stimulation therapy.

One of the conditions to which neural modulation can be applied to is obstructive sleep apnea (OSA), a respiratory disorder characterized by recurrent episodes of partial or complete obstruction of the upper airway during sleep. One of the causes of OSA is the inability of the tongue muscles to resist negative inspiratory pressure in the pharynx due to the sleep-related loss in muscle tone. As the tongue is pulled backwards, it obstructs the upper airway, decreasing ventilation and lowering lung and blood oxygen levels. Stimulation of the hypoglossal nerve and/or of the ansa cervicalis for example causes the tongue muscles, e.g. the genioglossus muscle, to contract, thereby maintaining an open, unobstructed airway as the genioglossus muscle is responsible for the forward movement of the tongue as well as for the stiffening of the anterior pharyngeal wall.

In order to detect, monitor and study the occurrence of sleep apnea events as well as to study the effectiveness of nerve stimulation and in particular hypoglossal or ansa cervicalis nerve stimulation (HGNS and ACS) as a way of treatment of sleep apnea, Polysomnography (PSG) is regularly be implemented. PSG allows for monitoring of many body functions, e.g. brain activity (EEG), eye movements (EOG), muscle activity or skeletal muscle activation (EMG), thermocouples, oxygen saturation and heart rhythm (ECG) etc. In addition, according to this disclosure, PSG data further comprises airflow data and respiratory bands signals. The aforementioned data can regularly be captured during any standard PSG-based method.

### Prior art

Studies on the effectiveness of sleep apnea treatment, or "Functional capacity evaluations" (FSE), are known in the prior art. They often rely on the reduction of an Apnea-Hypopnea Index (AHI) or of an Oxygen Desaturation Index (ODI) as an indicator of therapy effectiveness. However, these techniques require collection of long-time PSG data, wherein long time can mean more than two nights of sleep studies, one before the therapy as a baseline, and an additional night post treatment. Once the long-time PSG data is collected, the AHI/ODI scorings are compared to assess the effectiveness of the therapy. However, results obtained this way cannot be used to guide titration or prediction of a therapy response at the beginning of the night/therapy. Furthermore, the results are prone to high variability.

Another well-known method for measuring effectiveness of HGNS therapy relies on visual observation of tongue protrusion or any other anatomical change that may reflect an HGNS-induced airway opening. These methods have the disadvantage of being rather qualitative than quantitative, thus lacking reliability.

Other studies may be of invasive nature, including procedures such as drug-induced sleep endoscopy (DISE). This method is used in the beginning of the therapy to guide the titration and identify the therapeutic HGNS and/or ACS settings. The disadvantages of this method, however, are plenty: It is highly invasive, with risks of blockage or stoppage of breathing and allergic reactions. Secondly, since it is drug-induced, the results may not reflect the actual therapeutic level needed in regular sleep condition. Lastly, natural sleep is not precisely reproduced, especially with respect to body positioning, which may significantly impact the therapy response.

Further approaches known from the prior art include ultrasound techniques (Korotun et al., 2020, https://doi.org/10.1093/sleep/zsaa056.645; Al-Sherif et al., 2020, doi: 10.21037/jtd-cus-2020-001), tomographic techniques (Xiao et al., 2020, doi: 10.1177/0194599820901499) or baseline overnight sleep studies (Schwartz et al., 2011, doi: 10.1164/rccm.201109-1614OC).

US 2014/0228905 A1 is considered the closest prior art and discloses a method for stimulation of the hypoglossal nerve. The method comprises sensing a biological parameter indicative of respiration, analyzing the biological parameter to identify a respiratory cycle, identifying an inspiratory phase of that cycle and delivering a stimulation when the duration of the inspiratory phase exceeds a predetermined duration. The method comprises transferring stimulation parameter data and physiological data to a data processing unit, identifying physiological signals related to airway opening, and detecting breath cycles.

### Summary of the invention

One of the objectives of the present disclosure is to respond to the disadvantages of the prior art and to provide a method that enables short-time analysis of physiological data of sleep apnea patients treated with neurostimulation to quantify the efficacy of the applied therapy in different stimulation configurations and/or patient conditions. In order to do so, the method presented herein is a computer-implemented method. The current invention is defined by the appended claims.

According to one aspect of the invention, a computer-implemented method for quantification of a level of response to nerve stimulation is provided, the method comprising the following steps:
a) Transfer stored stimulation parameter data obtained during a previous stimulation therapy to a data processing unit
b) Transfer at least a subgroup of stored physiological data corresponding to a subject to the data processing unit;
c) Identify at least one physiological signal of the physiological data corresponding to the therapy's impact on the subject's airway opening, e.g. an airflow signal;
d) Detect ON-instants and OFF-instants in a defined time segment of the physiological data, wherein each ON-instant corresponds to an instant of time in which the subject was stimulated and wherein each OFF-instant corresponds to an instant of time in which the subject was not stimulated;
e) Detect at least one breath cycle (also referred to as "respiratory cycle" in this disclosure);
f) For each breath cycle detected, analyze an airflow segment and check if it was synchronized with an ON-instant or with an OFF-instant;
g) Sort each airflow segment into one of two groups based on its respectively checked synchronization with an ON/OFF-instant, the two groups being an ON-group comprising airflow signals that are synchronized with ON-instants and an OFF-group comprising airflow signals that are synchronized with OFF-instants;
h) For each of the two groups, separately determine an airflow curve from all airflow instants of each respective group, with respect to their relative occurrence within the breath cycle;
i) Calculate a volume of air for each of the two airflows;
j) Determine an impact of stimulation based on a ratio of the two volumes.

Nerve stimulation according to this disclosure may comprise stimulation or modulation of a nerve or of a plurality of nerves corresponding to the upper airway of a subject or patient, i.e. of a nerve or of a plurality of nerves that have an effect on the genioglossus muscle, especially for treatment of obstructive sleep apnea. In this regard, nerve stimulation may in particular refer to stimulation of the Hypoglossal nerve or of the Ansa Cervicalis nerves.

Advantageously, only a subgroup of the physiological data is required to carry out the method of this disclosure. A subgroup can for example comprise one or more subsets of the physiological data corresponding to a subject, e.g. only PSG or even only specific traces of the PSG data. This subgroup of the physiological data may then be transferred to the processing unit.

The physiological signal of step c) may be any type of electrical or non-electrical signal. Preferably, the physiological signal comprises PSG signal. However, the physiological signal can also be a live image or a sound.

The airflow curve determined in step h) may preferably be an average airflow curve. However, it is also possible that a mean airflow curve, a maximum airflow curve or a minimum airflow curve is determined. Likewise, the volume of air calculated in step i) may preferably be an average volume of air. However, it is also possible that a mean volume of air, a maximum volume of air or a minimum volume of air is calculated.

Furthermore, the method may comprise the steps:
k) Determine a therapy "hit rate" based on the percentage of breath inspirations (or a portion of them), within the defined time segment, synchronized with an ON-instant;
l) Determine the subject averaged respiratory rate within the defined time segment.

All relevant parameters of the neurostimulation to be evaluated are stored, for example as a data file, e.g. an *xls*-type data file (more commonly referred to as an Excel sheet). The data processing unit used with the presented computer-implemented method may then be programmed to automatically retrieve the respective parameters of interest from the xls-data file. These parameters may for example include pulse frequency, pulse duration, amplitude, ramp within train duration, train length, train interval of a stimulation etc. Furthermore, stimulation parameter data may include physiological parameters of the subject as well as the subject's body/head position and/or sleep stages.

The physiological data may preferably be PSG data, wherein PSG data may comprise g. brain activity (EEG), eye movements (EOG), muscle activity or skeletal muscle activation (EMG), thermocouples, oxygen saturation, heart rhythm (ECG), airflow data and respiratory bands signals. The physiological data and/or the PSG data may be stored as a *edf-type* data file. As is the case for the stimulation parameters input file, the data processing unit can be programmed to automatically retrieve the respective signals of interest included in the PSG data.

According to a beneficial embodiment, the method may comprise step
b') Superimpose the transferred physiological data and the transferred stimulation parameter data onto each other. This enables a user to split the physiological data into time segments of fixed stimulation configuration and subject condition;

The data processing unit may be part of a processor that is configured to perform all logical steps of the method presented herein. The processor may therefore include any electric circuit that may be configured to perform a logic operation on at least one input variable. The processor may include one or more integrated circuits, microchips, microcontrollers, and microprocessors, which may be all or part of a central processing unit (CPU), a digital signal processor (DSP), a field programmable gate array (FPGA), or any other circuit known to those skilled in the art that may be suitable for executing instructions or performing logic operations. According to a preferred embodiment, the processor is part of a computer or a handheld computing device.

The ON- and OFF-instants are defined as timings within the EMG signals of the PSG data in which the stimulation on or off, respectively. This step serves has the advantage that it allows for a quantification of a level of response to Hypoglossal or Ansa Cervicalis nerves stimulation.

The method presented can preferably be performed with a technician/FCE tool used to analyze and measure effectiveness of a neurostimulation therapy. It particularly allows for detection of the actual periods of neurostimulation of the subject based on EMG signals. Using the EMG signals, airflow signals can be analyzed breath-by-breath to quantify, among other things, a baseline airflow without HGNS and/or ACS, an airflow that was synchronized with HGNS and/or ACS, and subsequently the actual relative change in the subject's airflow as a result of the therapy applied.

Further to the above, the method presented enables to quantify the number of breaths and/or inspirations (or the average portion of inspirations) that are in synch with a stimulation event or, more accurately, that were in synch with a stimulation event during the stimulation performed earlier. The rate of breaths and/or inspirations that are in synch with the HGNS and/or ACS over the total number of breaths (i.e. number of breaths and/or inspirations that are in synch with the HGNS in addition to the of number breaths and/or inspirations that are out of synch with the HGNS) within a defined time segment, is called "hit rate". A combination of the above data provides a reliable method to determine the effectiveness of a given HGNS and/or ACS therapy. Therefore, the method is very useful for prediction of response to an applied HGNS and/or ACS therapy, thus enabling better and more precise of titration of the stimulation settings.

Furthermore, the presented method enables HGNS and/or ACS response assessment on any portion of the sleep, and more precisely on small time-segments, from a single night post-implantation, only based on non-invasive, passive data collection of EMG signals, airflow signals and respiratory bands signals, which can all be obtained from PSG data. This leads to prompt results.

In addition, the effectiveness of the analyzed therapy may be assessed directly based on the therapy's impact on the subject's airflow rather than only indirectly thought the quantification of a potential reduction of sleep apnea related symptoms (such as AHI, ODI etc.).

According to a preferred embodiment of the computer-implemented method for quantification of a level of response to nerve stimulation disclosed herein, the method may further comprise the following step:
c") Define at least one time segment within both transferred data, wherein step c") is preferably carried before step c).

Therefore, the method allows for precise quantification of HGNS and/or ACS effectiveness within a desired window of time of the PSG data from a subject. This window can be chosen to be very small, thus giving the method a clear time advantage over conventional PSG-based methods.

As a result, the FSE can be based on objective, quantitative and reliable measurements of therapy parameters, rather than on visual scoring of sleep apnea symptoms that can be highly subjective.

The method may further be characterized in that the ON-instants and the OFF-instants detected are each detected based on EMG signals of the PSG data. In addition, the at least one respiratory cycle may also be detected based on respiratory bands signals of the PSG data. According to a specific embodiment, at least 70 respiratory cycles may be detected based on respiratory bands signals of the physiological data. It is also possible that detection of respiratory cycles may be based on other signals of the physiological data, e.g. on signals obtained by motion or acoustic sensors.

The determined average airflow of the ON-group in step g) may in particular correspond to the average airflow induced by nerve stimulation, e.g. by stimulation of the hypoglossal nerve or of the Ansa Cervicalis nerves. Furthermore, the determined average airflow of the OFF-group in step g) may correspond to the average airflow without stimulation of the nerve.

It is also possible that a baseline is determined in step h) may be provided, the baseline corresponding to the average airflow determined within the OFF-group. Using the OFF-group as a baseline is beneficial, since it allows determination of the impact of the therapy without the need for a baseline PSG prior to stimulation treatment.

In addition, the calculation of the average volume of air inspired in synch with nerve stimulation may be based on an area under a graphed inspiration portion of the average airflow induced by nerve stimulation (A). Accordingly, the calculation of the average airflow without stimulation of the nerve may also be based on an area under a graphed inspiration portion of the average airflow without stimulation of the respective nerve (B).

In a preferred embodiment, the method may further comprise calculation of a stimulation hit rate. The stimulation hit rate may in particular comprise the following steps:
l) For each respiratory cycle, determine a percentage of an inspiration phase that was in synch with the respective nerve stimulation.
m) Calculate an overall hit rate as a percentage of respiratory cycles with more than a pre-defined threshold of their inspiration phase in synch with the respective nerve stimulation compared to respiratory cycles with less than said pre-defined threshold.

For example, if the hit rate = 80%, then this means that 80% of a certain amount of breaths had more than a predefined-threshold of their inspiration phase in synch with the HGNS and/or ACS.

Likewise, 20% had less than a predefined-threshold of their inspiration phase in synch with the HGNS and/or ACS.

Preferably, the percentage of the inspiration phase that as in synch with the respective nerve stimulation may be determined based on the detected ON- and/or OFF-instants.

According to a beneficial embodiment of the method, at least one of the following intermediate and/or final results may be displayed graphically:
- stimulation ON/OFF-instants
- respiratory/breath cycles
- airflow signals
- airflow
- volume of air
- impact of stimulation
- inspiration portion of the average airflow
- volume of air inspired with respective nerve stimulation
- volume of air inspired without stimulation of the respective nerve
- percentage of an inspiration phase that was in synch with the respective nerve stimulation
- average respiratory rate
- Hit Rate

The airflow curve determined in step h) may preferably be an average airflow curve. However, it is also possible that a mean airflow curve, a maximum airflow curve or a minimum airflow curve is determined. Likewise, the volume of air calculated in step i) may preferably be an average volume of air. However, it is also possible that a mean volume of air, a maximum volume of air or a minimum volume of air is calculated.

In particular, the above results may be displayed on a screen of a computer or a handheld electronic device, e.g. a smartphone. In a preferred embodiment, the results may be displayed using a specified program comprising a graphical user interface (GUI).

In particular, the method allows to use stored data, i.e. data that was obtained during a passed stimulation therapy.

Further to the above, the method further may then comprise the following step:
k) a nerve stimulation protocol is adjusted based on the impact of stimulation determined in step j).

Adjustment of the stimulation protocol can be carried out manually or automatically. This way, the method can also be used in a closed-loop manner, wherein the results obtained with the method are directly used to adjust stimulation of the subject. Thus, an intelligent or interactive method can be presented according to this disclosure.

The method presented herein can for example be used at early stages of a HGNS and/or ACS therapy to facilitate titration of the HGNS and/or ACS settings. Furthermore, the method can be used to predict therapy response on site during a titration. Alternatively or in to the above, the method can retrospectively on any PSG data recorded from HGNS and/or ACS patients (e.g. for research activities).

The invention is not limited to one of the embodiments described herein but may be modified in numerous other ways.

All features disclosed by the claims, the specifications and the figures, as well as all advantages, including constructive particulars, spatial arrangements and methodological steps, can be essential to the invention either on their own or by various combinations with each other.

### Brief description of exemplary embodiments

The accompanying drawings, which are incorporated in and constitute a part of this specification, illustrate several examples of the disclosed subject matter. The drawings show the following:
- Fig. 1a: a flowchart comprising steps of the method according to a preferred embodiment;
- Fig. 1b: a flowchart comprising steps of the method according to another embodiment;
- Fig. 2: the steps of Figs. 1a,b with examples of intermediate results;
- Fig. 3: the steps of Figs. 1a,b with examples of final results.

### Detailed description of exemplary embodiments

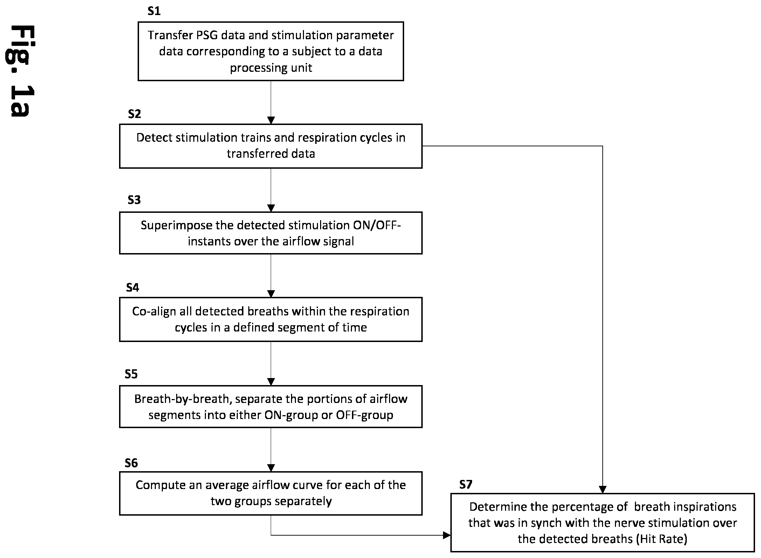

Fig. 1a depicts a flowchart comprising steps S1 to S7 of the method according to a preferred embodiment. The method shown in Fig. comprises a first step S1: Transfer physiological data (in case of the embodiment shown in Fig. 1a PSG data) and stimulation parameter data corresponding to a subject to a data processing unit (e.g. a computer). The parameter data may preferably be stored as an .x/s-file. The data processing unit used with the presented computer-implemented method may then be programmed to automatically retrieve the respective parameters of interest from the data file. The physiological data is preferably stored as an *.edf-file.* Edf-files are the standardize digital format of any PSG study. Likewise, the data processing unit can be programmed to automatically retrieve the respective signals of interest included in the physiological data.

After both data have been transferred to the data processing unit, a processor automatically detects stimulation trains (i.e. the ON/OFF-instants) and respiration cycles in the transferred data according to a second step S2. This way, ON-instants and OFF-instants within the physiological data are detected. The ON- and OFF-instants are defined as timings within the EMG signals of the PSG data in which the stimulation on or off, respectively.

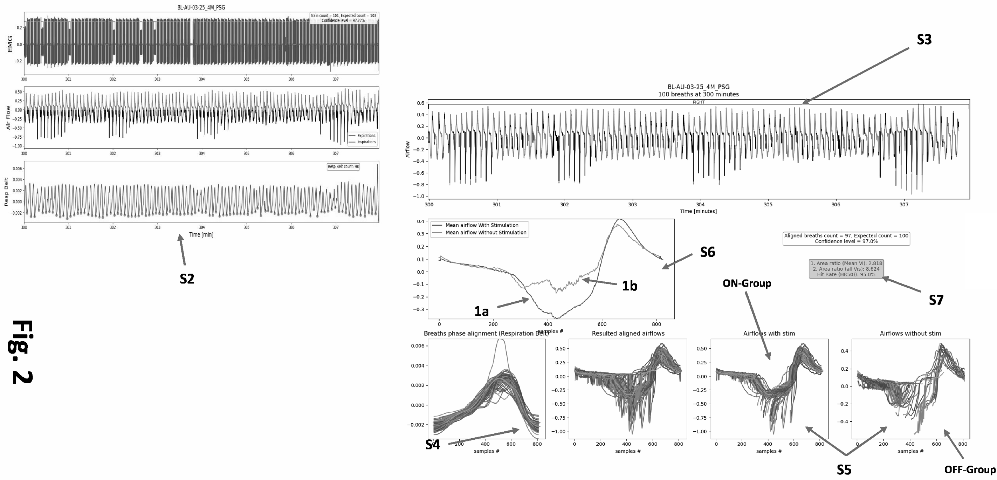

As shown in Fig. 1a and Fig. 2, S2 may be followed by a third step S3, according to which the detected stimulation trains (i.e. the ON/OFF-instants) are superimposed over the airflow signal retrieved from the physiological data. Preferably, this step is also automatically performed by the processor of the computer used to perform the method implemented thereon. After superimposing stimulation trains on the airflow signal, the superimposed data may be color coded for improved visualization, as is shown in Fig. 2.

After S3, a fourth step S4 is performed: Co-align all detected breaths within the respiration cycles in a defined segment of time.

After the ON- and OFF-instants are detected and the detected breaths are co-aligned, a step S5 follows: Separate, breath-by-breath, portions of airflow segments into either ON-group or OFF-group , wherein the ON-group comprises airflow signals that are synchronized with ON-instants and wherein the OFF-group comprises airflow signals that are synchronized with OFF-instants.

Based on the two groups, a step S6 is performed, during which an average airflow curve is computed for each of the two groups separately. These curves, corresponding either to airflow signals of an average single breath cycle, that are synchronized with ON-instants or OFF-instants (depending on the curve being computed for either the ON-group or the OFF-group), comprising an inspiration portion 1a, 1b (see Fig. 2). Based on an area under the inspiration portion of each of the average airflow curves 1a, 1b a respective average volume of air inspired either in synch with nerve stimulation or out of synch with nerve stimulation can be calculated.

Lastly, a step S7 may be performed: Determine the percentage of breath inspirations that were in synch with the nerve stimulation over the detected breaths (Hit Rate).

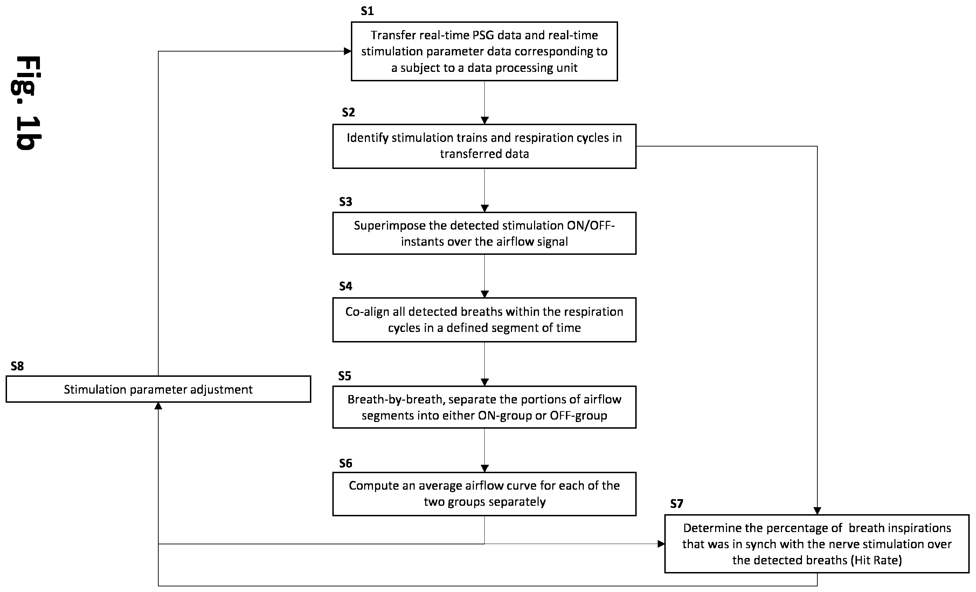

The method of Fig. 1b differs from the method of Fig. 1a in that Fig. 1b depicts a closed-loop method. In particular, the transferred data used for method of Fig. 1b is obtained in real-time, i.e. during or in between stimulation of a subject sessions or patient (see step S1).

Once the method produces results, e.g. in form of a hit rate, the stimulation parameters used for stimulation of a subject can be adjusted based on those results. This can happen either manually or automatically.

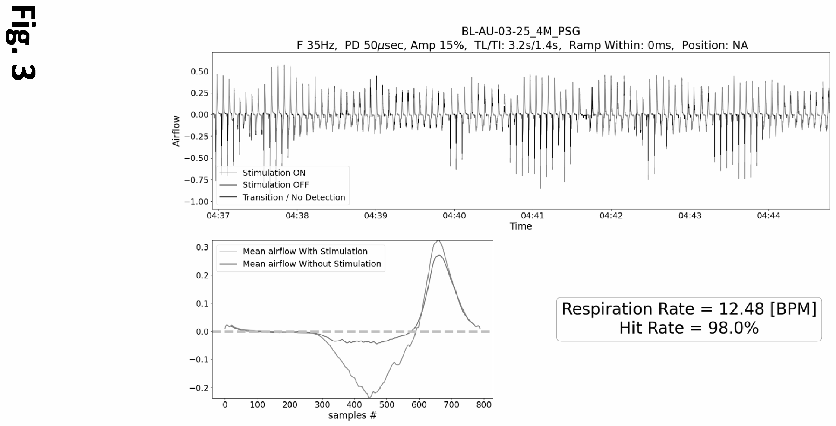

Figs. 2 and 3 show intermediate results (Fig. 2) and final results (Fig. 3) as preferably displayed by a suitable device.

## Claims

1. Computer-implemented method for quantification of a level of response to nerve stimulation, the method comprising the following steps:
a) Transfer (S1) a stored stimulation parameter data obtained during a previous stimulation therapy to a data processing unit;
b) transfer (S1) at least a subgroup of stored physiological data obtained during a previous stimulation therapy corresponding to a subject to the data processing unit;
c) identify (S2) at least one physiological signal of the physiological data corresponding to the therapy's impact on the subject's airway opening;
d) detect (S2) ON-instants and OFF-instants in a defined time segment of the physiological data, wherein each ON-instant corresponds to an instant of time in which the subject was stimulated and wherein each OFF-instant corresponds to an instant of time in which the subject was not stimulated;
e) detect (S2) at least one breath cycle;
f) for each breath cycle detected, analyze (S5) an airflow segment and check if it was synchronized with an ON-instant or with an OFF-instant;
g) sort (S5) each airflow segment into one of two groups based on its respectively checked synchronization with an ON/OFF-instant, the two groups being an ON-group comprising airflow signals that are synchronized with ON-instants and an OFF-group comprising airflow signals that are synchronized with OFF-instants;
h) for each of the two groups, separately determine (S6) an airflow curve (1a, 1b) from all airflow segments of each respective group;
i) calculate (S6) an volume of air for each of the two airflows;
j) determine (S6) an impact of stimulation based on visualization of the two airflows and/or on a ratio of the two volumes.

2. Method of claim 1 **characterized in that** the physiological data comprises PSG data.

3. Method of any of the preceding claims, **characterized in that** the method further comprises the following step:
c') superimpose (S3) the transferred physiological data and the transferred stimulation parameter data onto each other, wherein step c') is preferably carried out before step c).

4. Method of any of the preceding claims, **characterized in that** the method further comprises the following step:
c") define at least one time segment within both transferred data, wherein step c") is preferably carried out before step c').

5. Method of any of the preceding claims, **characterized in that** the method further comprises the following step:
d') co-align (S4) all detected breath cycles within respiration cycles in the defined time segment.

6. Method of any of the preceding claims, **characterized in that** the ON-instants and the OFF-instants detected are each detected based on EMG signals of the physiological data.

7. Method of any of the preceding claims, **characterized in that** the at least one breath cycle is detected based on respiratory bands signals of the physiological data.

8. Method of any of the preceding claims, **characterized in that** the determined average airflow of the ON-group in step i) corresponds to the average airflow induced by nerve stimulation (A) and that the determined average airflow of the OFF-group in step i) corresponds to the average airflow without stimulation of the nerve (B).

9. Method of any of the preceding claims, **characterized in that** a baseline is determined in step i), the baseline corresponding to the average airflow determined within the OFF-group.

10. Method of any of the preceding claims, **characterized in that** the method further comprises the following step:
k) calculate a volume of air inspired with nerve stimulation and an average airflow without stimulation of the nerve.

11. Method of claim 10 wherein the calculation of the volume of air inspired with nerve stimulation is based on an area under a graphed inspiration portion of the airflow induced by nerve stimulation (A); and
wherein the calculation of the average airflow without stimulation of the nerve is based on an area under a graphed inspiration portion of the airflow without stimulation of the nerve (B).

12. Method of any of the preceding claims, **characterized in that** the method further comprises calculation of a stimulation hit rate.

13. Method of claim 12, **characterized in that** the calculation of a stimulation hit rate comprises the following steps:
I) for each breath cycle, determine a percentage of an inspiration phase that was in synch with the nerve stimulation, pre-defining a desired threshold;
m) calculate an overall hit rate as a percentage of breath cycles with more than the pre-defined threshold of their inspiration phase in synch with the nerve stimulation compared to breath cycles with less than said pre-defined threshold.

14. Method of claim 13, **characterized in that** the percentage of the inspiration phase that as in synch with the nerve stimulation is determined based on the detected ON- and/or OFF-instants.

15. Method of any of the preceding claims, **characterized in that** at least one of the following intermediate and/or final results is graphically displayed:
- stimulation ON/OFF-instants
- breath cycles
- airflow signals
- average airflow
- average volume of air
- impact of stimulation
- inspiration portion of the airflow
- volume of air inspired with nerve stimulation
- volume of air inspired without stimulation of the nerve
- hit rate
- average respiratory rate

## Patentansprüche

1. Computerimplementiertes Verfahren zum Quantifizieren eines Grades der Reaktion auf eine Nervenstimulation, wobei das Verfahren die folgenden Schritte umfasst:
a) Übertragen (S1) von gespeicherten Stimulationsparameterdaten, die während einer früheren Stimulationstherapie erhalten wurden, an eine Datenverarbeitungseinheit;
b) Übertragen (S1) mindestens einer Teilgruppe von gespeicherten physiologischen Daten, die während einer vorherigen Stimulationstherapie entsprechend einem Subjekt erhalten wurden, an die Datenverarbeitungseinheit;
c) Identifizieren (S2) mindestens eines physiologischen Signals der physiologischen Daten, das der Wirkung der Therapie auf die Öffnung der Atemwege des Subjekts entspricht;
d) Erkennen (S2) von AN-Zeitpunkten und AUS-Zeitpunkten in einem definierten Zeitsegment der physiologischen Daten, wobei jeder AN-Zeitpunkt einem Zeitpunkt entspricht, zu dem das Subjekt stimuliert wurde, und wobei jeder AUS-Zeitpunkt einem Zeitpunkt entspricht, zu dem das Subjekt nicht stimuliert wurde;
e) Erkennen (S2) mindestens eines Atemzyklus;
f) für jeden erkannten Atemzyklus Analysieren (S5) eines Luftstromsegments und Prüfen, ob es mit einem AN-Zeitpunkt oder mit einem AUS-Zeitpunkt synchronisiert war;
g) Sortieren (S5) jedes Luftstromsegments in eine von zwei Gruppen basierend auf seiner jeweilig geprüften Synchronisation mit einem AN-/Aus-Zeitpunkt, wobei es sich bei den zwei Gruppen um eine AN-Gruppe, die Luftstromsignale umfasst, die mit AN-Zeitpunkten synchronisiert sind, und eine AUS-Gruppe, die Luftstromsignale umfasst, die mit AUS-Zeitpunkten synchronisiert sind, handelt;
h) für jede der zwei Gruppen separates Bestimmen (S6) einer Luftstromkurve (1a, 1b) aus allen Luftstromsegmenten jeder jeweiligen Gruppe;
i) Berechnen (S6) eines Luftvolumens für jeden der zwei Luftströme;
j) Bestimmen (S6) einer Wirkung der Stimulation basierend auf einer Visualisierung der zwei Luftströme und/oder einem Verhältnis der zwei Volumina.

2. Verfahren nach Anspruch 1, **dadurch gekennzeichnet, dass** die physiologischen Daten PSG-Daten umfassen.

3. Verfahren nach einem der vorhergehenden Ansprüche, **dadurch gekennzeichnet, dass** das Verfahren ferner den folgenden Schritt umfasst:
c') Übereinanderlegen (S3) der übertragenen physiologischen Daten und der übertragenen Stimulationsparameterdaten, wobei Schritt c') vorzugsweise vor Schritt c) ausgeführt wird.

4. Verfahren nach einem der vorhergehenden Ansprüche, **dadurch gekennzeichnet, dass** das Verfahren ferner den folgenden Schritt umfasst:
c") Definieren mindestens eines Zeitsegment in beiden übertragenen Daten, wobei Schritt c") vorzugsweise vor Schritt c') ausgeführt wird.

5. Verfahren nach einem der vorhergehenden Ansprüche, **dadurch gekennzeichnet, dass** das Verfahren ferner den folgenden Schritt umfasst:
d') Miteinander-Ausrichten (S4) aller erkannten Atemzyklen innerhalb von Respirationszyklen in dem definierten Zeitsegment.

6. Verfahren nach einem der vorhergehenden Ansprüche, **dadurch gekennzeichnet, dass** die AN-Zeitpunkte und die AUS-Zeitpunkte jeweils basierend auf EMG-Signalen der physiologischen Daten erkannt werden.

7. Verfahren nach einem der vorhergehenden Ansprüche, **dadurch gekennzeichnet, dass** der mindestens eine Atemzyklus basierend auf Atmungsbandsignalen der physiologischen Daten erkannt wird.

8. Verfahren nach einem der vorhergehenden Ansprüche, **dadurch gekennzeichnet, dass** der bestimmte durchschnittliche Luftstrom der AN-Gruppe in Schritt i) dem durchschnittlichen Luftstrom entspricht, der durch die Nervenstimulation (A) induziert wird, und dass der bestimmte durchschnittliche Luftstrom der AUS-Gruppe in Schritt i) dem durchschnittlichen Luftstrom ohne Stimulation des Nervs (B) entspricht.

9. Verfahren nach einem der vorhergehenden Ansprüche, **dadurch gekennzeichnet, dass** in Schritt i) eine Grundlinie bestimmt wird, wobei die Grundlinie dem durchschnittlichen Luftstrom entspricht, der in der AUS-Gruppe bestimmt wird.

10. Verfahren nach einem der vorhergehenden Ansprüche, **dadurch gekennzeichnet, dass** das Verfahren ferner den folgenden Schritt umfasst:
k) Berechnen eines Volumens der bei Nervenstimulation eingeatmeten Luft und eines durchschnittlichen Luftstroms ohne Stimulation des Nervs.

11. Verfahren nach Anspruch 10, wobei die Berechnung des Volumens der bei Nervenstimulation eingeatmeten Luft auf einer Fläche unter einem graphisch dargestellten Einatmungsabschnitt des durch Nervenstimulation (A) induzierten Luftstroms basiert; und
wobei die Berechnung des durchschnittlichen Luftstroms ohne Stimulation des Nervs auf einer Fläche unter einem graphisch dargestellten Einatmungsabschnitt des Luftstroms ohne Stimulation des Nervs (B) basiert.

12. Verfahren nach einem der vorhergehenden Ansprüche, **dadurch gekennzeichnet, dass** das Verfahren ferner die Berechnung einer Stimulationstrefferquote umfasst.

13. Verfahren nach Anspruch 12, **dadurch gekennzeichnet, dass** die Berechnung einer Stimulationstrefferquote die folgenden Schritte umfasst:
I) für jeden Atemzyklus Bestimmen eines Prozentsatzes einer Einatmungsphase, die mit der Nervenstimulation synchron war, wodurch ein gewünschter Schwellenwert vordefiniert wird;
m) Berechnen einer Gesamttrefferquote als einen Prozentsatz von Atemzyklen mit mehr als dem vordefinierten Schwellenwert ihrer Einatmungsphase synchron mit der Nervenstimulation, im Vergleich zu Atemzyklen mit weniger als dem vordefinierten Schwellenwert.

14. Verfahren nach Anspruch 13, **dadurch gekennzeichnet, dass** der Prozentsatz der Einatmungsphase, der synchron mit der Nervenstimulation ist, basierend auf den erkannten AN- und/oder AUS-Zeitpunkten bestimmt wird.

15. Verfahren nach einem der vorhergehenden Ansprüche, **dadurch gekennzeichnet, dass** mindestens eines der folgenden Zwischen- und/oder Endergebnisse graphisch angezeigt werden:
- Stimulations-AN-/Aus-Zeitpunkte
- Atemzyklen
- Luftstromsignale
- durchschnittlicher Luftstrom
- durchschnittliches Luftvolumen
- Auswirkung der Stimulation
- Einatmungsabschnitt des Luftstroms
- Volumen der bei Nervenstimulation eingeatmeten Luft
- Volumen der ohne Stimulation des Nervs eingeatmeten Luft
- Trefferquote
- durchschnittliche Respirationsrate

## Revendications

1. Procédé mis en œuvre par ordinateur permettant de quantifier un niveau de réponse à une stimulation nerveuse, le procédé comprenant les étapes suivantes :
a) le transfert (S1) de données de paramètres de stimulation stockées obtenues lors d'une thérapie de stimulation précédente à une unité de traitement de données ;
b) le transfert (S1) d'au moins un sous-groupe de données physiologiques stockées obtenues lors d'une thérapie de stimulation précédente correspondant à un sujet à l'unité de traitement de données ;
c) l'identification (S2) d'au moins un signal physiologique des données physiologiques correspondant à l'impact de la thérapie sur l'ouverture des voies respiratoires du sujet ;
d) la détection (S2) d'instants ON et d'instants OFF dans un segment temporel défini des données physiologiques, dans lequel chaque instant ON correspond à un instant pendant lequel le sujet a été stimulé et chaque instant OFF correspond à un instant pendant lequel le sujet n'a pas été stimulé ;
e) la détection (S2) d'au moins un cycle respiratoire ;
f) pour chaque cycle respiratoire détecté, l'analyse (S5) d'un segment de flux d'air et le fait de vérifier s'il était synchronisé avec un instant ON ou avec un instant OFF ;
g) le tri (S5) de chaque segment de flux d'air dans l'un de deux groupes sur la base de sa synchronisation vérifiée respectivement avec un instant ON/OFF, les deux groupes étant un groupe ON comprenant des signaux de flux d'air qui sont synchronisés avec des instants ON et un groupe OFF comprenant des signaux de flux d'air qui sont synchronisés avec des instants OFF ;
h) pour chacun des deux groupes, la détermination de manière séparée (S6) d'une courbe de flux d'air (1a, 1b) à partir de tous les segments de flux d'air de chaque groupe respectif ;
i) le calcul (S6) d'un volume d'air pour chacun des deux flux d'air ;
j) la détermination (S6) d'un impact de stimulation sur la base de la visualisation des deux flux d'air et/ou sur un rapport des deux volumes.

2. Procédé selon la revendication 1, **caractérisé en ce que** les données physiologiques comprennent des données PSG.

3. Procédé selon l'une quelconque des revendications précédentes, **caractérisé en ce que** le procédé comprend en outre l'étape suivante :
c') la superposition (S3) des données physiologiques transférées et des données de paramètres de stimulation transférées les unes sur les autres, dans lequel l'étape c') est effectuée de préférence avant l'étape c).

4. Procédé selon l'une quelconque des revendications précédentes, **caractérisé en ce que** le procédé comprend en outre l'étape suivante :
c") la définition d'au moins un segment temporel au sein des deux données transférées, dans lequel l'étape c") est effectuée de préférence avant l'étape c').

5. Procédé selon l'une quelconque des revendications précédentes, **caractérisé en ce que** le procédé comprend en outre l'étape suivante :
d') le co-alignement (S4) de tous les cycles respiratoires détectés au sein des cycles respiratoires dans le segment temporel défini.

6. Procédé selon l'une quelconque des revendications précédentes, **caractérisé en ce que** les instants ON et les instants OFF détectés sont chacun détectés sur la base de signaux EMG des données physiologiques.

7. Procédé selon l'une quelconque des revendications précédentes, **caractérisé en ce que** l'au moins un cycle respiratoire est détecté sur la base de signaux de bandes respiratoires des données physiologiques.

8. Procédé selon l'une quelconque des revendications précédentes, **caractérisé en ce que** le flux d'air moyen déterminé du groupe ON à l'étape i) correspond au flux d'air moyen induit par stimulation nerveuse (A) et **en ce que** le flux d'air moyen déterminé du groupe OFF à l'étape i) correspond au flux d'air moyen sans stimulation du nerf (B).

9. Procédé selon l'une quelconque des revendications précédentes, **caractérisé en ce qu'**une ligne de base est déterminée à l'étape i), la ligne de base correspondant au flux d'air moyen déterminé dans le groupe OFF.

10. Procédé selon l'une quelconque des revendications précédentes, **caractérisé en ce que** le procédé comprend en outre l'étape suivante :
k) le calcul d'un volume d'air inspiré avec stimulation nerveuse et un flux d'air moyen sans stimulation du nerf.

11. Procédé selon la revendication 10, dans lequel le calcul du volume d'air inspiré par stimulation nerveuse est basé sur une zone située sous une partie d'inspiration graphique du flux d'air induit par stimulation nerveuse (A) ; et
dans lequel le calcul du flux d'air moyen sans stimulation du nerf est basé sur une zone située sous une partie d'inspiration graphique du flux d'air sans stimulation du nerf (B).

12. Procédé selon l'une quelconque des revendications précédentes, **caractérisé en ce que** le procédé comprend en outre le calcul d'un taux de réussite de stimulation.

13. Procédé selon la revendication 12, **caractérisé en ce que** le calcul d'un taux de réussite de stimulation comprend les étapes suivantes :
l) pour chaque cycle respiratoire, la détermination d'un pourcentage d'une phase d'inspiration qui était synchronisée avec la stimulation nerveuse, en prédéfinissant un seuil souhaité ;
m) le calcul d'un taux de réussite global, exprimé en pourcentage, des cycles respiratoires dont la phase d'inspiration est synchronisée avec la stimulation nerveuse au-delà du seuil prédéfini, par rapport aux cycles respiratoires dont la phase d'inspiration est synchronisée en deçà dudit seuil prédéfini.

14. Procédé selon la revendication 13, **caractérisé en ce que** le pourcentage de la phase d'inspiration qui est synchronisé avec la stimulation nerveuse est déterminé sur la base des instants ON et/ou OFF détectés.

15. Procédé selon l'une quelconque des revendications précédentes, **caractérisé en ce qu'**au moins l'un des résultats intermédiaires et/ou finaux suivants est affiché graphiquement :
- instants ON/OFF de la stimulation
- cycles respiratoires
- signaux de flux d'air
- flux d'air moyen
- volume d'air moyen
- impact de la stimulation
- partie d'inspiration du flux d'air
- volume d'air inspiré avec stimulation nerveuse
- volume d'air inspiré sans stimulation du nerf
- taux de réussite
- fréquence respiratoire moyenne
